# EUROPEAN PATENT APPLICATION

(11) **EP 4 501 947 A1**
(43) Date of publication of application: **05.02.2025**
(21) Application number: 23781006.4
(22) Date of filing: 30.03.2023
(51) Int. Cl.: C07K 14/165, A61K 39/02, A61K 39/155, A61K 39/215, A61P 31/04, A61P 31/14, C07K 14/11, C07K 14/135, C07K 14/315, C07K 19/00, C12N 9/26, C12N 15/31, C12N 15/44, C12N 15/45, C12N 15/50, C12N 15/62

(54) **FUSION PROTEIN AND VACCINE**

(30) Priority: 31.03.2022 JP 2022057799
(71) Applicant: The Research Foundation for Microbial Diseases of Osaka University, Suita-shi, Osaka 565-0871 (JP)
(72) Inventor: YOSHIOKA, Yasuo, Suita-shi, Osaka 565-0871 (JP)
(74) Representative: Müller-Boré & Partner Patentanwälte PartG mbB
(86) International application number: PCT/JP2023/013408
(87) International publication number: WO 2023/190998

(57) **Abstract**

The present invention provides a fusion protein which is useful as a vaccine antigen against infectious diseases. A fusion protein including (a) a combination of hemagglutinin and an N-terminal domain of SARS-CoV-2, (b) a combination of PspA and a receptor binding domain of SARS-CoV-2, (c) a combination of hemagglutinin and respiratory syncytial virus G protein, or (d) a combination of PspA and hemagglutinin, is useful as a vaccine antigen against infectious diseases.

## Description

### Technical Field

The present invention relates to a fusion protein useful as a vaccine antigen against infectious diseases and a vaccine using the same.

### Background Art

Unlike the conventional injectable vaccines, nasal vaccines are expected as a next-generation vaccine that can protect the infection itself, such as inducing antibody production even in the upper respiratory tract, which is an initial infection site of many pathogens including novel coronavirus.

On the other hand, it is difficult for the nasal vaccine to induce an immune response sufficient for infection protection by single administration of a vaccine antigen, and thus combined use with an adjuvant is supposed to be indispensable. Therefore, studies on the adjuvant have been actively conducted in order to enhance the effectiveness of the nasal vaccine (for example, NPL 1 to 5).

### Citation List

### Non Patent Literatures

NPL 1: The Journal of the Japanese Association for Infectious Diseases 71 (2), 153 to 161, 1997
NPL 2: Scientific Reports volume 6, Article number: 29165 (2016)
NPL 3: Journal of Dental Health 67: 2 to 10, 2017
NPL 4: Inflammation and immunity 27 (2): 99 to 102 2019
NPL 5: Expert Opinion on Drug Metabolism & Toxicology Volume 16, 2020 - Issue 11 p. 1051 to 1061

### Summary of Invention

### Technical Problem

Intranasal IgA not only binds to and eliminates pathogens, but also transports the pathogens into the nasal lymphatic tissue through transcytosis, to efficiently induce antigen-specific immune responses. Therefore, the present inventor has considered that, by fusing a target vaccine antigen to a protein (carrier protein) recognized by IgA present in the nasal cavity, IgA itself becomes an antigen delivery carrier (carrier antibody) and delivers the vaccine antigen to the nasal lymphatic tissue, thereby making it possible to induce mucosal immunity efficiently.

In view of the above, an object of the present invention is to provide a fusion protein which is useful as a vaccine antigen against infectious diseases.

### Solution to Problem

As a result of intensive studies, the present inventor has found that a fusion protein containing (a) a combination of an N-terminal domain of a coronavirus and hemagglutinin and/or neuraminidase, (b) a combination of a receptor binding domain of a coronavirus and pneumococcal surface protein A (PspA), (c) a combination of a G protein of an RS virus and hemagglutinin and/or neuraminidase, or (d) a combination of PspA and hemagglutinin and/or neuraminidase is useful as a vaccine antigen against infectious diseases. The present invention has been completed by further conducting studies based on this finding.

Item 1. A fusion protein containing:
(a) a combination of an N-terminal domain of a coronavirus and hemagglutinin and/or neuraminidase; (b) a combination of a receptor binding domain of a coronavirus and PspA; (c) a combination of a G protein of an RS virus and hemagglutinin and/or neuraminidase; or (d) a combination of PspA and hemagglutinin and/or neuraminidase.

Item 2. The fusion protein according to item 1, wherein the fusion protein contains the combination (a) or the combination (b), and is used as a vaccine antigen against a coronavirus.

Item 3. The fusion protein according to item 1, wherein the fusion protein contains the combination (c), and is used as a vaccine antigen against an RS virus.

Item 4. The fusion protein according to item 1, wherein the fusion protein contains the combination (d), and is used as a vaccine antigen against Streptococcus pneumoniae.

Item 5. A vaccine containing the fusion protein according to any one of items 1 to 4.

Item 6. The vaccine according to item 5, wherein the vaccine does not contain an adjuvant.

Item 7. The vaccine according to item 5 or 6, wherein the vaccine contains the combination (a), the combination (c), or the combination (d), and is administered to a subject having an antibody against an influenza virus.

Item 8. The vaccine according to item 5 or 6, wherein the vaccine contains the combination (b), and is administered to a subject having an antibody against Streptococcus pneumoniae.

Item 9. The vaccine according to any one of items 5 to 8, wherein the vaccine is nasally administered.

Item 10. An immunization method including administering, to a subject having an antibody against an influenza virus, a vaccine containing a fusion protein containing: (a) a combination of an N-terminal domain of a coronavirus and hemagglutinin and/or neuraminidase; (c) a combination of a G protein of an RS virus and hemagglutinin and/or neuraminidase; or (d) a combination of PspA and hemagglutinin and/or neuraminidase.

Item 11. An immunization method including administering, to a subject having an antibody against Streptococcus pneumoniae, a vaccine containing a fusion protein containing (b) a combination of a receptor binding domain of a coronavirus and PspA. Advantageous Effects of Invention

According to the present invention, there is provided a fusion protein which is useful as a vaccine antigen against infectious diseases.

### Brief Description of Drawings

[Fig. 1] Fig. 1 shows results of induction of NTD-specific IgG in plasma and results of induction of NTD-specific IgA in the nasal cavity when a fusion protein containing the combination (a) obtained in Test Example 1 is used for a vaccine antigen.
[Fig. 2] Fig. 2 shows results of induction of RBD-specific IgG in plasma and results of induction of RBD-specific IgA in the nasal cavity when a fusion protein containing the combination (b) obtained in Test Example 2 is used for a vaccine antigen.
[Fig. 3] Fig. 3 shows results of induction of G protein-specific IgG in plasma and results of induction of G protein-specific IgA in the nasal cavity when a fusion protein containing the combination (c) obtained in Test Example 3 is used for a vaccine antigen.
[Fig. 4] Fig. 4 shows results of induction of PspA-specific IgG in plasma and results of induction of PspA-specific IgA in the nasal cavity when a fusion protein containing the combination (d) obtained in Test Example 4 is used for a vaccine antigen.
[Fig. 5] Fig. 5 shows results of body weight change and survival rate when a fusion protein containing the combination (d) obtained in Test Example 5 is used for a vaccine antigen.

### Description of Embodiments

### 1. Fusion protein

The fusion protein of the present invention contains an antigen (hereinafter, also described as "target antigen") of a virus and/or a bacterium to be a target for infection prevention and an antigen (hereinafter, also described as "carrier protein") of an antibody that a human to be vaccinated has in the body. Specifically, the fusion protein of the present invention contains (a) a combination of an N-terminal domain of a coronavirus and hemagglutinin and/or neuraminidase, (b) a combination of a receptor binding domain of a coronavirus and PspA, (c) a combination of a G protein of an RS virus and hemagglutinin and/or neuraminidase, or (d) a combination of PspA and hemagglutinin and/or neuraminidase.

Which of the two proteins contained in the combinations (a) to (d) is designed as a target antigen and which is designed as a carrier protein is not particularly limited. In a preferred embodiment, the target antigen can be designed to be an N-terminal domain (NTD) of a coronavirus in the combination (a), a receptor binding domain (RBD) of a coronavirus in the combination (b), a G protein of an RS virus in the combination (c), and PspA in the combination (d). That is, in the preferred embodiment, the carrier protein can be designed to be hemagglutinin and/or neuraminidase in the combinations (a), (c), and (d), and PspA in the combination (b).

### 1-1. Target antigen

The coronavirus from which the target antigens used in the combinations (a) and (b) are derived is morphologically spherical with a diameter of about 100 to 200 nm, and has protrusions on the surface thereof. The coronavirus is classified into the order of Nidovirales, the subfamily of Coronavirinae, the family of Coronavirinae. In the envelope of the lipid bilayer membrane, there is a genome of positive-stranded single-stranded RNA wound around a nucleocapsid protein (also referred to as "nucleocapsid"), and a spike protein (hereinafter, also referred to as "spike"), an envelope protein (hereinafter, also referred to as "envelope"), and a membrane protein are arranged on the surface of the envelope. The size of the viral genome is about 30 kb, the longest among RNA viruses.

Coronaviruses are classified into groups of alpha, beta, gamma, and delta according to genetic characteristics. As coronaviruses infecting humans, four types of human coronaviruses 229E, OC43, NL63, and HKU-1 as causative viruses of cold, and severe acute respiratory syndrome (SARS) coronavirus that occurred in 2002 and Middle East respiratory syndrome (MERS) coronavirus that occurred in 2012, which cause serious pneumonia, are known. Human coronaviruses 229E and NL63 are classified into the alpha coronavirus genus, and human coronaviruses OC43, HKU-1, SARS coronavirus, and MERS coronavirus are classified into the beta coronavirus genus. In the present invention, the coronavirus is preferably a coronavirus belonging to the beta coronavirus genus, and more preferably a SARS coronavirus.

SARS-CoV-2 classified as SARS coronavirus has been isolated and identified as a causative virus of the novel coronavirus infection that occurred in Wuhan in 2019. SARS-CoV-2 has been mutated repeatedly from the early Wuhan strain, and mutant strains such as a strain detected in the United Kingdom, a strain detected in South Africa, and a strain detected in India have been found. There is also a possibility that a mutant strain that has not yet been detected or a new mutant strain will occur in the future. Therefore, in the present invention, the SARS coronavirus is not limited to the above-described SARS-CoV-2 strains, and includes other SARS-CoV-2 mutant strains newly detected in the future.

The N-terminal domain (NTD) of SARS-CoV-2 used in the combination (a) is a region of the N-terminal of the amino acid of the spike protein (S protein) and has a binding target site for the infectivity-enhancing antibody, and NTDs are crosslinked by the infectivity-enhancing antibody. More specifically, when the infectivity-enhancing antibody binds to NTD, the conformation of RBD is changed to a more open conformation as compared with a case where the infectivity-enhancing antibody does not bind to NTD, thereby controlling RBD to more easily bind to angiotensin converting enzyme 2 (ACE2) (Cell, Volume 184, Issue 13, 24 June 2021, Pages 3452 to 3466).

Examples of the NTD of SARS-CoV-2 include the following (1-1) to (1-3):
(1-1) a polypeptide consisting of an amino acid sequence from position 1 to 303 of SEQ ID NO: 1;
(1-2) a polypeptide in which one or several amino acid residues are substituted, added, inserted and/or deleted in an amino acid sequence from position 1 to 303 of SEQ ID NO: 1 and that forms a complex so that the polypeptides are crosslinked by an infectivity-enhancing antibody; and
(1-3) a polypeptide that has a sequence identity of 80% or more with an amino acid sequence from position 1 to 303 of SEQ ID NO: 1 and forms a complex such that the polypeptides are crosslinked by an infectivity-enhancing antibody.

In the polypeptide of (1-1), SEQ ID NO: 1 represents an amino acid sequence of a spike protein (YP_009724390.1) of SARS-CoV-2 listed as NC_045512 in NCBI, and an amino acid sequence from position 1 to 303 thereof corresponds to the NTD.

The RBD of SARS-CoV-2 used in the combination (b) is a region having a binding site in the spike protein of SARS-CoV-2 with ACE2.

The RBD of SARS-CoV-2 includes the following (2-1) to (2-3):
(2-1) a polypeptide consisting of an amino acid sequence from position 319 to 541 of SEQ ID NO: 1;
(2-2) a polypeptide in which one or several amino acid residues are substituted, added, inserted and/or deleted in an amino acid sequence from position 319 to 541 of SEQ ID NO: 1 and that forms a complex with ACE2; and
(2-3) a polypeptide that has a sequence identity of 80% or more with an amino acid sequence from position 319 to 541 of SEQ ID NO: 1 and forms a complex with ACE2.

In the polypeptide of (2-1), SEQ ID NO: 1 represents an amino acid sequence of a spike protein (YP_009724390.1) of SARS-CoV-2 listed as NC_045512 in NCBI, and an amino acid sequence from position 319 to 541 thereof corresponds to the RBD, as described in the polypeptide of (1-1).

The RS virus from which the target antigen used in the combination (c) is derived causes RS virus infection such as infantile acute respiratory tract infection (bronchiolitis, pneumonia, and the like). The G protein of the RS virus contributes to adhesion to the host cell surface. In the present invention, it is preferable to use a conserved region among regions of the G protein of the RS virus.

Examples of the conserved region of the G protein of the RS virus include the following (3-1) to (3-3):
(3-1) a polypeptide consisting of an amino acid sequence from position 161 to 197 of SEQ ID NO: 2;
(3-2) a polypeptide in which one or several amino acid residues are substituted, added, inserted and/or deleted in an amino acid sequence from position 161 to 197 of SEQ ID NO: 2 and that adheres to a host cell surface; and
(3-3) a polypeptide that has a sequence identity of 80% or more with an amino acid sequence from position 161 to 197 of SEQ ID NO: 2 and adheres to a host cell surface.

In the polypeptide of (3-1), SEQ ID NO: 2 represents an amino acid sequence of a G protein of an RS virus listed in UniProtKB-P03423.

The PspA of Streptococcus pneumoniae used in the combination (d) exhibits pathogenicity due to its complement inhibitory activity of inhibiting binding of a complement C3 to the pneumococcal bacterial cell surface.

Examples of the PspA include the following (4-1) to (4-3):
(4-1) a polypeptide consisting of an amino acid sequence from position 32 to 409 of SEQ ID NO: 3;
(4-2) a polypeptide in which one or several amino acid residues are substituted, added, inserted, and/or deleted in an amino acid sequence from position 32 to 409 of SEQ ID NO: 3 and that inhibits binding of a complement factor C3 to Streptococcus pneumoniae; and
(4-3) a polypeptide that has a sequence identity of 80% or more with an amino acid sequence from position 32 to 409 of SEQ ID NO: 3 and inhibits binding of a complement factor C3 to Streptococcus pneumoniae.

In the polypeptide of (4-1), SEQ ID NO: 3 represents an amino acid sequence of a PspA listed in GenBank as Accession No. AF071814.

The polypeptides of (1-1) to (1-3), (2-1) to (2-3), (3-1) to (3-3), and (4-1) to (4-3) include not only polypeptides obtained by artificial substitution, but also polypeptides originally having such amino acid sequences.

The polypeptides of (1-2) and (1-3) are allowed to have a difference in amino acid sequence as compared with the polypeptide of (1-1) as long as they have an ability to form a complex in which the polypeptides are crosslinked by an infectivity-enhancing antibody, as in the polypeptide of (1-1), and are also referred to as a different form of the polypeptide of (1-1). The polypeptides of (2-2) and (2-3) are allowed to have a difference in amino acid sequence as compared with the polypeptide of (2-1) as long as they have an ability to form a complex with ACE2, as in the polypeptide of (2-1), and are also referred to as a different form of the polypeptide of (2-1). The polypeptides of (3-2) and (3-3) are allowed to have a difference in amino acid sequence as compared with the polypeptide of (3-1) as long as they have an ability to adhere to the host cell surface, as in the polypeptide of (3-1), and are also referred to as a different form of the polypeptide of (3-1). The polypeptides of (4-2) and (4-3) are allowed to have a difference in amino acid sequence as compared with the polypeptide of (4-1) as long as they have an ability to inhibit binding of a complement factor C3 to Streptococcus pneumoniae, as in the polypeptide of (4-1), and are also referred to as a different form of the polypeptide of (4-1).

The difference in amino acid in the polypeptides of (1-2), (2-2), (3-2), and (4-2) may include only one difference (for example, substitution) among substitution, addition, insertion, and deletion, or may include two or more differences (for example, substitution and insertion). In the polypeptides of (1-2), (2-2), (3-2), and (4-2), the number of amino acid differences may be 1 or several, and is, for example, 1 to 15, preferably 1 to 10, more preferably 1 to 8, still more preferably 1 to 7 or 1 to 6, still even more preferably 1 to 5 or 1 to 4, still further more preferably 1 to 3, particularly preferably 1 or 2, and most preferably 1.

The sequence identity between each of the amino acid sequences of the polypeptides of (1-3), (2-3), (3-3), and (4-3) and each of the amino acid sequences set forth in (1-1), (2-1), (3-1), and (4-1) may be 80% or more, and is preferably 85% or more, more preferably 90% or more, still more preferably 95% or more, still even more preferably 96% or more, still further more preferably 97% or more, particularly preferably 98% or more, 99% or more, or 99.5% or more.

Here, in the polypeptides of (1-3), (2-3), (3-3), and (4-3), the "sequence identity" indicates a value of identity of an amino acid sequence obtained by a bl2seq program (Tatiana A. Tatsusova, Thomas L. Madden, FEMS Microbiol. Lett., Vol. 174, p. 247 to 250, 1999) of BLAST PACKAGE [sgi32 bit edition, Version 2.0.12; available from National Center for Biotechnology Information (NCBI)]. The parameters may be set to Gap insertion Cost value: 11 and Gap extension Cost value: 1.

When an amino acid substitution is introduced into the polypeptides of (1-2), (1-3), (2-2), (2-3), (3-2), (3-3), (4-2), and (4-3), conservative substitution is mentioned as an aspect of the amino acid substitution. That is, in the polypeptides of (1-2), (1-3), (2-2), (2-3), (3-2), (3-3), (4-2), and (4-3), examples of the amino acid substitution to be introduced into the amino acid sequences set forth in SEQ ID NO: 1 to 3 include substitution with another non-polar amino acid if the amino acid before substitution is a non-polar amino acid, substitution with another uncharged amino acid if the amino acid before substitution is an uncharged amino acid, substitution with another acidic amino acid if the amino acid before substitution is an acidic amino acid, and substitution with another basic amino acid if the amino acid before substitution is a basic amino acid.

### 1-2. Carrier protein

The carrier protein is an antigen that constitutes the fusion protein of the present invention, and is an antigen against an antibody that a subject to be vaccinated has in the body. Specifically, the carrier protein is an antigen against an antibody that the subject has in the body due to past infection or vaccination. The carrier protein may be an antigen against an antibody that the subject has in the body. Examples of the carrier protein include influenza virus, RS virus, Streptococcus pneumoniae, and coronavirus. Specifically, the carrier protein is fused with a target antigen in each of the combinations (a) to (d), whereby the fusion protein of the present invention is provided with or improved in a property of inducing a specific antibody against an antigen of a virus and/or a bacteria to be a target for infection prevention and/or onset prevention (hereinafter, also described as "target antigen-specific antibody inducing property").

Among the hemagglutinins and/or neuraminidases used in the combinations (a), (c) and (d), from the viewpoint of providing the fusion protein of the present invention with a further improved target antigen-specific antibody inducing property, hemagglutinin is preferred, hemagglutinin derived from influenza virus is more preferred, hemagglutinin derived from influenza A virus (H1 type hemagglutinin to H16 type hemagglutinin) or hemagglutinin derived from influenza B virus is still more preferred, H1 type, H3 type, H5 type, or H7 type hemagglutinin is still even more preferred, H1 type or H3 type hemagglutinin is further more preferred, and H1 type hemagglutinin is most preferred. The amino acid sequences of these hemagglutinins and neuraminidases can be obtained from the official database.

Preferred amino acid sequences of the hemagglutinin used in the combinations (a), (c) and (d) include the following (5-1) to (5-3):
(5-1) a polypeptide consisting of an amino acid sequence from position 18 to 523 of SEQ ID NO: 4;
(5-2) a polypeptide in which one or several amino acid residues are substituted, added, inserted and/or deleted in an amino acid sequence from position 18 to 523 of SEQ ID NO: 4 and that improves a target antigen-specific antibody inducing property; and
(5-3) a polypeptide that has a sequence identity of 80% or more with an amino acid sequence from position 18 to 523 of SEQ ID NO: 4 and improves a target antigen-specific antibody inducing property.

In the polypeptide of (5-1), SEQ ID NO: 4 represents an amino acid sequence of a H1 subunit of influenza A H1N1 type (A/California/04/2009) listed as ACV82259.1 in NCBI, and an amino acid sequence from position 18 to 523 obtained by excluding the leader sequence of the N-terminal portion and the extracellular domain of the C-terminal portion is used as the amino acid sequence of the polypeptide of (5-1).

Examples of the amino acid sequence of the PspA used in the combination (b) include the following (6-1) to (6-3):
(6-1) a polypeptide consisting of an amino acid sequence from position 32 to 409 of SEQ ID NO: 3;
(6-2) a polypeptide in which one or several amino acid residues are substituted, added, inserted and/or deleted in an amino acid sequence from position 32 to 409 of SEQ ID NO: 3 and that improves a target antigen-specific antibody inducing property; and
(6-3) a polypeptide that has a sequence identity of 80% or more with an amino acid sequence from position 32 to 409 of SEQ ID NO: 3 and improves a target antigen-specific antibody inducing property.

In the polypeptide of (6-1), SEQ ID NO: 3 represents an amino acid sequence of a PspA listed in GenBank as Accession No. AF071814, as described in the polypeptide of (4-1).

The polypeptides of (5-1) to (5-3) and (6-1) to (6-3) include not only polypeptides obtained by artificial substitution, but also polypeptides originally having such an amino acid sequence.

The polypeptides of (5-2), (5-3), (6-1), and (6-2) are allowed to have a difference in amino acid sequence as compared with the polypeptides of (5-1) and (6-1) as long as they improve the target antigen-specific antibody titer inducing property, and are also referred to as a different form of the polypeptides of (5-1) and (6-1). The phrase "to improve the target antigen-specific antibody inducing property" means that the target antigen-specific antibody titer in a case where a fusion protein is formed together with the above target antigen is made higher than the specific antibody titer in a case where the target antigen is used alone. In a preferred aspect of "to improve the target antigen-specific antibody titer inducing property" in the above (5-2), (5-3), (6-1), and (6-2), the target antigen-specific antibody titer when a fusion protein is formed together with the target antigen is equal to or higher than that in a case where the polypeptides of the above (5-1) and (6-1) are fused, more preferably, when the target antigen-specific antibody titer that is induced in a case where the polypeptides of the above (5-1) and (6-1) are fused with the target antigen is 100%, for example, about 80 to 120% or 90 to 110%.

The difference in amino acid in the polypeptides of (5-2) and (6-2) may include only difference (for example, substitution) among substitution, addition, insertion, and deletion, or may include two or more differences (for example, substitution and insertion). In the polypeptide of (2-2), the number of amino acid differences may be 1 or several, and is, for example, 1 to 15, preferably 1 to 10, more preferably 1 to 8, still more preferably 1 to 7 or 1 to 6, still even more preferably 1 to 5 or 1 to 4, still further more preferably 1 to 3, particularly preferably 1 or 2, and most preferably 1.

The sequence identity in the above (5-3) and (6-3) is the same as the sequence identity described in the above (1-3) to (4-3).

The aspect of the amino acid substitution when an amino acid substitution is introduced into the polypeptides of (5-2), (5-3), (6-1), and (6-2) is the same as the aspect of the amino acid substitution in the polypeptides of (1-2) to (4-2) and (1-3) to (4-3).

The fusion protein of the present invention may have other structures in addition to the target antigen and the carrier protein. Examples of other structures include linker peptides that bind the target antigen and the carrier protein. Examples of the linker peptide include linker peptides consisting of the following amino acid sequences. The linker peptide is, for example, preferably GS linkers such as Linker Example 1 and Linker Example 2, more preferably Linker Example 1, and still more preferably a linker in which n = 1 to 3 (preferably 1 to 2, more preferably 1) and m = 1 in Linker Example 1.

| | |
|---|---|
| Linker Example 1: [(G)ₙS]ₘ | n = 1 to 5, m = 1 to 5 |
| Linker Example 2: [(G)ₙS]ₘGG | n = 1 to 5, m = 1 to 5 |
| Linker Example 3: (G)ₙ | n = 5 to 10 |
| Linker Example 4: (EAAAK)ₙ | n = 1 to 4 |
| Linker Example 5: (PA)ₙ | n = 2 to 34 |

The fusion peptide of the present invention can provide or improve a property of inducing a specific antibody against an antigen of a virus and/or a bacterium to be a target for infection prevention, as described above, and thus can be used as a vaccine antigen against the virus and/or the bacterium to be a target for infection prevention.

### 2. Method for producing fusion protein

The method for producing the fusion protein described in the above "1. Fusion protein" includes: a step 1 of introducing a DNA encoding a target antigen and a DNA encoding a carrier protein in each of the combinations (a) to (d) into a host cell to obtain a transformant; a step 2 of culturing the transformant; and a step 3 of recovering a product containing the target antigen and the carrier protein from the cultured transformant.

In the step 1, the DNA encoding a target antigen to be a target for infection prevention and a DNA encoding a carrier protein can be introduced by inserting these DNAs into an expression vector in an appropriate form, and incorporating the obtained recombinant vector into a host cell. Preferably, a recombinant vector in which a DNA encoding a target antigen to be a target for infection prevention, a DNA encoding a linker peptide, and a DNA encoding a carrier protein are incorporated in this order can be produced and introduced into a host cell.

In the method for designing and synthesizing DNA, base sequence information of each gene of a target polypeptide, specifically, a target antigen and a carrier protein is acquired from a known database (for example, GenBank) or the like; a target gene is cloned using a known method; base sequence information is acquired by performing base sequence analysis; a nucleic acid is extracted from a microorganism having a target antigen or a carrier protein based on the obtained base sequence information; and a gene is acquired using a known means such as PCR, thereby making it possible to produce a fusion gene (hybrid gene) in which the respective genes are artificially linked.

When the target polypeptide has a mutation, a mutation is introduced into a gene encoding the polypeptide. A method for introducing a specific mutation into a specific site of a base sequence are known, and for example, site-directed mutation introduction of DNA, site-directed mutagenesis, and the like (specifically, a Kunkel method, a Gapped duplex method, a megaprimer PCR method, and the like) can be used.

The above DNA is preferably one in which the codon usage frequency is mainly optimized for a host, and more preferably one in which the codon usage frequency is optimized for a mammalian cell. As an index representing the codon usage frequency, a total of the host optimal codon usage frequency of each codon may be adopted. The optimal codon is defined as a codon having the highest usage frequency among codons corresponding to the same amino acid.

The recombinant vector is obtained by inserting the above DNA into an appropriate expression vector by restriction enzyme cleavage, ligation, or the like. The expression vector is not particularly limited, and those constructed for genetic recombination from phage, plasmid, or virus capable of autonomously growing in a host are suitable.

The recombinant vector includes a control factor such as a promoter operably linked to the above DNA. The control factor typically includes a promoter, but may further include a transcription element such as an enhancer, a CCAAT box, a TATA box, or an SPI site as necessary. In addition, operably linking means that various control factors such as a promoter and an enhancer that control the above DNA are linked to the above DNA in a state in which they can operate in a host cell.

The host is not particularly limited as long as the recombinant vector is stable and can autonomously grow and express the trait of an exogenous gene. Suitable examples of the host include bacteria belonging to the genus Escherichia such as Escherichia coli, the genus Bacillus such as Bacillus subtilis, and the genus Pseudomonas such as Pseudomonas putida; and yeasts, but the host may also be animal cells such as 293 cells, 293T cells, Expi293F cells, and CHO cells, insect cells, plant cells, and the like. Among them, Escherichia coli is particularly preferable.

Conditions for introducing the recombinant vector into a host cell in order to obtain a transformant may be appropriately set according to the type of the host cell and the like. Examples of the method using a bacteria as a host cell include a method using a competent cell treated by calcium ion treatment and an electroporation method. Examples of the method in the case of using a yeast as a host cell include an electroporation method, a spheroplast method, and a lithium acetate method. Examples of the method in the case of using an animal cell as a host cell include an electroporation method, a calcium phosphate method, and a lipofection method. Examples of the method in the case of using an insect cell as a host cell include a calcium phosphate method, a lipofection method, and an electroporation method. Examples of the method in the case of using a plant cell as a host cell include an electroporation method, an Agrobacterium method, a particle gun method, and a PEG method.

Whether or not the recombinant vector is incorporated into a host cell can be confirmed by a known method such as a PCR method, a Southern hybridization method, or a Northern hybridization method.

In the step 2, a fusion protein containing a target antigen and a carrier protein can be produced as a product by culturing a transformant.

The culture conditions of the transformant may be appropriately set in consideration of the nutritional physiological properties of the host, and liquid culture is preferable. In the case of industrial production, aeration stirring culture is preferable. As a nutrient source of the medium, those required for growth of the transformant can be used. The culture temperature can be appropriately set within a range in which the transformant can grow and the transformant produces the fusion protein.

In the step 3, the product is recovered. The culture may be a culture supernatant, or a cultured cell, a cultured bacterial cell, or a disrupted product thereof. When a target fusion protein (product) is produced in a host cell, the product can be extracted by disrupting the host cell. When a target fusion protein (product) is produced outside the host cell, a culture solution can be used as it is, or the host cell can be removed by centrifugation or the like. Thereafter, the product can be recovered by isolation and/or purification using a general biochemical method used for isolation and purification of proteins, for example, ammonium sulfate precipitation, gel filtration, ion exchange chromatography, affinity chromatography, or the like alone or in appropriate combination.

### 3. Vaccine

Since the fusion protein of the present invention can provide or improve a property of inducing a specific antibody against a target antigen as described above, and is useful as a vaccine antigen against a virus and/or a bacterium to be a target for infection prevention and/or onset prevention, the present invention also provides a vaccine containing the above fusion protein.

The vaccine of the present invention may contain other components such as a buffer, a tonicity agent, a soothing agent, a preservative, and an antioxidant depending on the purpose, use, and the like, in addition to the fusion protein as a vaccine antigen.

Examples of the buffer include buffers such as phosphate, acetate, carbonate, and citrate. Examples of the tonicity agent include sodium chloride, glycerin, and D-mannitol. Examples of the soothing agent include benzyl alcohol. Examples of the preservative include thimerosal, para-hydroxybenzoates, phenoxyethanol, chlorobutanol, benzyl alcohol, phenethyl alcohol, dehydroacetic acid, sorbic acid, antibiotics, and synthetic antibacterial agents. Examples of the antioxidant include sulfite and ascorbic acid.

In addition to the above components, the vaccine of the present invention may also contain a light-absorbing dye (riboflavin, adenine, adenosine, and the like), a stabilizer (a chelating agent, a reducing agent, and the like), a carbohydrate (sorbitol, lactose, mannitol, starch, sucrose, glucose, dextran, and the like), casein digest, various vitamins, a flavoring agent, an adjuvant, and the like.

Since the fusion protein of the present invention described above can provide or improve a specific antibody titer inducing property against a target antigen, the specific antibody titer inducing property can be effectively induced even when no adjuvant is blended in the vaccine. Therefore, a preferred aspect of the vaccine of the present invention includes those containing no adjuvant.

Furthermore, in the vaccine of the present invention, one or more vaccine antigens against a microorganism that causes another disease may be blended in addition to the above fusion protein as a vaccine antigen against a virus and/or a bacterium to be a target for infection prevention and/or onset prevention.

The dosage form of the vaccine of the present invention is not particularly limited, and can be appropriately determined based on the administration method, storage conditions, and the like. Specific examples of the dosage form include liquid preparations and solid preparations, and more specifically, oral administration agents such as tablets, capsules, powders, granules, pills, solutions, and syrups; and parenteral administration agents such as injections, sprays, application agents, and patches.

The method for administering the vaccine of the present invention is not particularly limited, and examples thereof include injection administration such as intramuscular, intraperitoneal, intradermal, and subcutaneous administration, inhalation administration from the nasal cavity and the oral cavity, and oral administration. As described above, the vaccine of the present invention does not require an adjuvant, and is particularly preferably administered by inhalation administration from the nasal cavity, that is, by nasal administration.

The subject to which the vaccine of the present invention is applied may be a subject that is to receive infection prevention and/or onset prevention against a virus and/or a bacteria, and that has an antibody against a virus and/or a bacteria different from the virus and/or bacteria to be a target of the infection prevention and/or onset prevention in the body thereof. Examples of animals as such a subject to which the vaccine of the present invention is applied include mammals, and more specifically, humans; pet animals such as dogs and cats; and experimental animals such as rats, mice, hamsters, and monkeys. In particular, in humans, since many of them have a history of vaccination with influenza and/or a history of infection with influenza, the vaccine of the present invention has high versatility for humans.

The dose of the vaccine of the present invention is not particularly limited, and can be appropriately determined according to the type of the active ingredient, the administration method, and the administration subject (conditions such as age, body weight, sex, and presence or absence of underlying disease).

The vaccine of the present invention can be used for the purpose of, for example, preventing the onset of an infectious disease caused by a virus and/or a bacterium to be a target for infection prevention, preventing and minimizing symptoms and pathological conditions associated with the infectious disease, and treating and reducing the symptoms and pathological conditions, and preferably, the vaccine is used for the purpose of the prevention and minimization described above.

### Examples

Hereinafter, the present invention will be described in detail with reference to Examples, but the present invention is not limited thereto. The amino acid sequence configuration of the fusion protein used in the following Test Examples is as follows.

**[Table 1]**

| | | |
|---|---|---|
| Target antigen | N-terminal domain of S protein derived from novel coronavirus (SARS-CoV-2 NTD) | Position 1 to 303 of SEQ ID NO: 1 |
| Target antigen | Receptor binding domain of S protein derived from novel coronavirus (SARS-CoV-2 RED) | Position 319 to 541 of SEQ ID NO: 1 |
| Target antigen | Conserved region of G protein derived from RS virus | Position 161 to 197 of SEQ ID NO: 2 |
| Target antigen | Pneumococcal surface protein A (PspA) | Position 32 to 409 of SEQ ID NO: 3 |
| Linker peptide | [(G)ₙS]ₘ | n = 1, m = 1 |
| Carrier protein | Hemagglutinin (HA) derived from A/California/7/2009 (Cal7) | Position 18 to 523 of SEQ ID NO: 4 |
| Carrier protein | Pneumococcal surface protein A (PspA) | Position 32 to 409 of SEQ ID NO: 3 |

### Test Example 1

### (1) Experimental animals, reagents, and the like

· Mouse: 6 week old male BALB/c mouse
· Infection virus: H1N1 influenza A virus (strain: A/California/7/2009 (Cal7))
· Vaccine antigen: As a fusion protein containing the combination (a), a fusion recombinant protein (NTD-HA) in which an N-terminal domain (NTD) of an S protein derived from a novel coronavirus (SARS-CoV-2) and hemagglutinin (HA) derived from Cal7 were linked by a linker peptide was prepared by a conventional method.

### (2) Method

An N-terminal domain (NTD) of an S protein derived from a novel coronavirus was used as a target antigen, and NTD-HA, which is a fusion protein of NTD and HA, was used as a vaccine antigen. The upper respiratory tracts of mice were previously infected with Cal7 to thereby create a situation in which the mice have an antibody against an influenza virus as well as adults. Specifically, the upper respiratory tracts of mice were infected with Cal7, and 30 days and 51 days after infection, the mice were nasally immunized with NTD-HA (Cal7-NTD-HA). Unvaccinated mice were used as controls (Cont). In addition, mice that were not infected with Cal7 and were nasally immunized with NTD-HA were also prepared (NTD-HA). As a solvent for the preparation for nasal administration, phosphate buffered saline (PBS) was used. Blood was collected 44 days after infection (14 days after the first immunization) and 58 days after infection (7 days after the second immunization), and NTD-specific IgG in plasma was evaluated by ELISA. Furthermore, nasal cavity wash specimens were collected 65 days after infection (14 days after the second immunization), and NTD-specific IgA in the nasal cavity was evaluated by ELISA.

### (3) Results

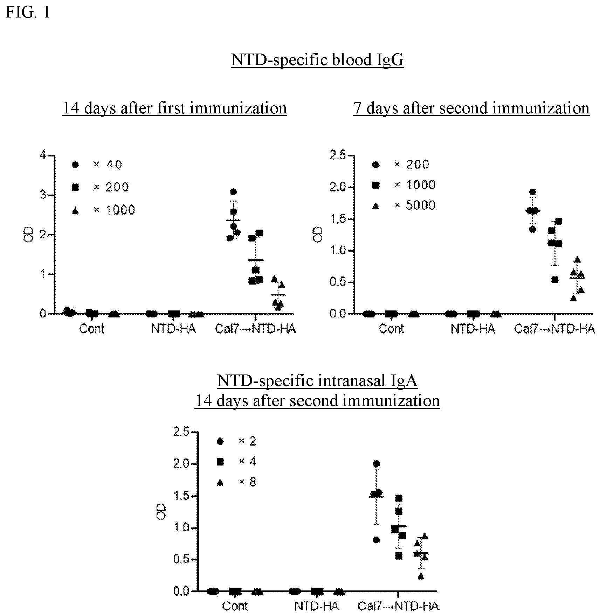

The results of induction of NTD-specific IgG in plasma and the results of induction of NTD-specific IgA in the nasal cavity are shown in Fig. 1. The magnification shown in Fig. 1 indicates the dilution rate of the plasma or the dilution rate of the nasal cavity wash specimen (the same applies to the following graphs). As shown in Fig. 1, when mice were nasally immunized with NTD-HA after infection with Cal7, NTD-specific blood IgG and NTD-specific intranasal IgA were observed without using an adjuvant. On the other hand, when mice having no history of infection with influenza virus were nasally immunized with NTD-HA, no antibody titer was observed.

### Test Example 2

### (1) Experimental animals, reagents, and the like

· Mouse: 6 week old male BALB/c mouse
· Infecting bacteria: Streptococcus pneumoniae (Sp)
· Vaccine antigen: As a fusion protein containing the combination (b), a fusion recombinant protein (RBD-PspA) in which a receptor binding domain (RBD) of an S protein derived from a novel coronavirus (SARS-CoV-2) and PspA derived from Streptococcus pneumoniae were linked by a linker peptide was prepared by a conventional method.

### (2) Method

A receptor binding domain (RBD) of an S protein derived from a novel coronavirus was used as a target antigen, and RBD-PspA, which is a fusion protein of RBD and PspA, was used as a vaccine antigen. The upper respiratory tracts of mice were previously infected with Streptococcus pneumoniae to thereby create a situation in which the mice have an antibody against Streptococcus pneumoniae. Specifically, the upper respiratory tracts of mice were infected with Streptococcus pneumoniae, and 30 days and 51 days after infection, the mice were nasally immunized with RBD or RBD-PspA (Sp→RBD or Sp→RBD-PspA, respectively). Mice infected with Streptococcus pneumoniae but not vaccinated were used as controls (Sp→PBS). In addition, mice that were not infected with Streptococcus pneumoniae and were nasally immunized with RBD-PspA were also prepared (RBD-PspA). As a solvent for the preparation for nasal administration, phosphate buffered saline (PBS) was used. Blood was collected 44 days after infection (14 days after the first immunization) and 58 days after infection (7 days after the second immunization), and RBD-specific IgG in plasma was evaluated by ELISA. Furthermore, nasal cavity wash specimens were collected 65 days after infection (14 days after the second immunization) and RBD-specific IgA in the nasal cavity was evaluated by ELISA.

### (3) Results

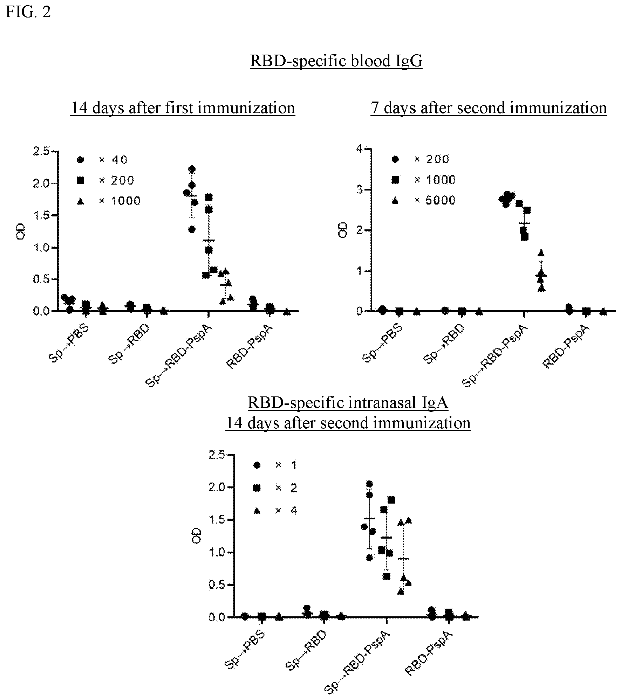

The results of induction of RBD-specific IgG in plasma and the results of induction of RBD-specific IgA in the nasal cavity are shown in Fig. 2. As shown in Fig. 2, when mice were nasally immunized with RBD-PspA after infection with Streptococcus pneumoniae, RBD-specific blood IgG and RBD-specific intranasal IgA were observed without using an adjuvant. On the other hand, when mice having no history of infection with Streptococcus pneumoniae were nasally immunized with RBD-PspA, no antibody titer was observed.

### Test Example 3

### (1) Experimental animals, reagents, and the like

· Mouse: 6 week old male BALB/c mouse
· Infection virus: H1N1 influenza A virus (strain: A/California/7/2009 (Cal7))
· Vaccine antigen: As a fusion protein containing the combination (c), a fusion recombinant protein (G-HA) in which a conserved region of a G protein derived from an RS virus and hemagglutinin (HA) derived from Cal7 were linked by a linker peptide was prepared by a conventional method.

### (2) Method

A G protein derived from an RS virus was used as a target antigen, and G-HA, which is a fusion protein of a G protein conserved region and HA, was used as a vaccine antigen. The upper respiratory tracts of mice were previously infected with Cal7 to thereby create a situation in which the mice have an antibody against an influenza virus as well as adults. Specifically, the upper respiratory tracts of mice were infected with Cal7, and 30 days and 51 days after infection, the mice were nasally immunized with G-HA (Cal7-G-HA). Unvaccinated mice were used as controls (Cont). In addition, mice that were not infected with Cal7 and were nasally immunized with G-HA were also prepared (G-HA). As a solvent for the preparation for nasal administration, phosphate buffered saline (PBS) was used. Blood was collected 44 days after infection (14 days after the first immunization) and 58 days after infection (7 days after the second immunization), and G protein-specific IgG in plasma was evaluated by ELISA. Furthermore, nasal cavity wash specimens were collected 65 days after infection (14 days after the second immunization), and G protein-specific IgA in the nasal cavity was evaluated by ELISA.

### (3) Results

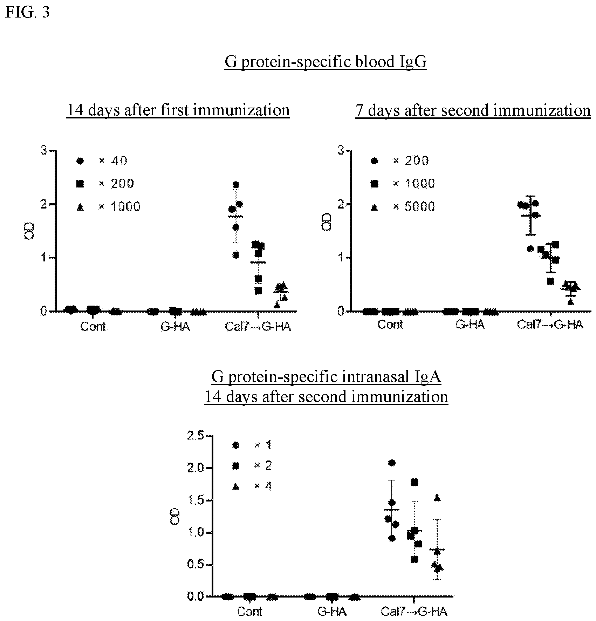

The results of induction of G protein-specific IgG in plasma and the results of induction of G protein-specific IgA in the nasal cavity are shown in Fig. 3. As shown in Fig. 3, when mice were nasally immunized with G-HA after infection with Cal7, G protein-specific blood IgG and G protein-specific intranasal IgA were observed without using an adjuvant. On the other hand, when mice having no history of infection with influenza virus were nasally immunized with G-HA, no antibody titer was observed.

### Test Example 4

### (1) Experimental animals, reagents, and the like

· Mouse: 6 week old male BALB/c mouse
· Infection virus: H1N1 influenza A virus (strain: A/California/7/2009 (Cal7))
· Vaccine antigen: As a fusion protein containing the combination (d), a fusion recombinant protein (PspA-HA) in which PspA derived from Streptococcus pneumoniae and hemagglutinin (HA) derived from Cal7 were linked by a linker peptide was prepared by a conventional method.

### (2) Method

PspA derived from Streptococcus pneumoniae was used as a target antigen, and PspA-HA, which is a fusion protein of PspA and HA, was used as a vaccine antigen. The upper respiratory tracts of mice were previously infected with Cal7 to thereby create a situation in which the mice have an antibody against an influenza virus as well as adults. Specifically, the upper respiratory tracts of mice were infected with Cal7, and 30 days and 51 days after infection, the mice were nasally immunized with PspA-HA (Cal7→PspA-HA). Unvaccinated mice were used as controls (Cont). In addition, mice that were not infected with Cal7 and were nasally immunized with PspA-HA were also prepared (PspA-HA). As a solvent for the preparation for nasal administration, phosphate buffered saline (PBS) was used. Blood was collected 44 days after infection (14 days after the first immunization) and 58 days after infection (7 days after the second immunization), and PspA-specific IgG in plasma was evaluated by ELISA. Furthermore, nasal cavity wash specimens were collected 65 days after infection (14 days after the second immunization) and PspA-specific IgA in the nasal cavity was evaluated by ELISA.

### (3) Results

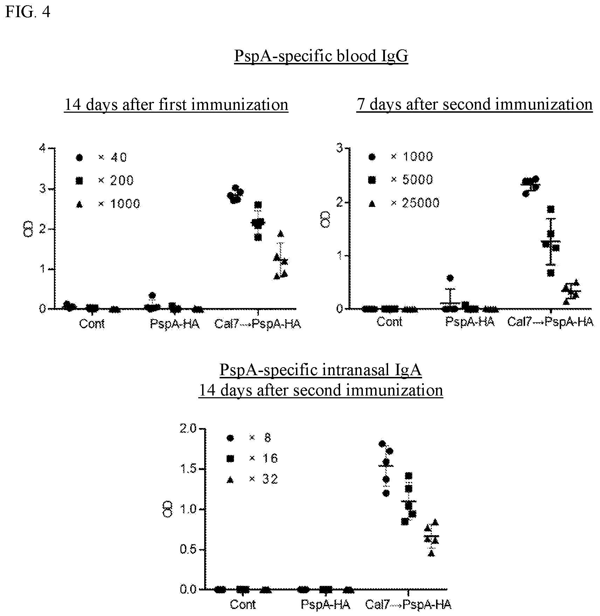

The results of induction of PspA-specific IgG in plasma and the results of induction of PspA-specific IgA in the nasal cavity are shown in Fig. 4. As shown in Fig. 4, when mice were nasally immunized with PspA-HA after infection with Cal7, PspA-specific blood IgG and PspA-specific intranasal IgA were observed without using an adjuvant. On the other hand, when mice having no history of infection with influenza virus were nasally immunized with PspA-HA, no antibody titer was observed.

### Test Example 5

### (1) Experimental animals, reagents, and the like

· Mouse: 6 week old male BALB/c mouse
· Infection virus: H1N1 influenza A virus (strain: A/California/7/2009 (Cal7))
· Infecting bacteria: Streptococcus pneumoniae (target antigen: PspA protein derived from Streptococcus pneumoniae)
· Vaccine antigen: As a fusion protein containing the combination (d), a fusion recombinant protein (PspA-HA) in which a PspA protein derived from Streptococcus pneumoniae and hemagglutinin (HA) derived from Cal7 were linked by a linker peptide was prepared by a conventional method.

### (2) Method

PspA derived from Streptococcus pneumoniae was used as a target antigen, and PspA-HA, which is a fusion protein of PspA and HA, was used as a vaccine antigen. The upper respiratory tracts of mice were previously infected with Cal7 to thereby create a situation in which the mice have an antibody against an influenza virus as well as adults. Specifically, the upper respiratory tracts of mice were infected with Cal7, and 30 days and 51 days after infection, the mice were nasally immunized with PspA-HA (flu→PspA-HA). Unvaccinated mice were used as controls (flu→PBS). In addition, mice that were not infected with Cal7 and were nasally immunized with PspA-HA were also prepared (no flu infection→PspA-HA). The lower respiratory tracts of mice were infected with Streptococcus pneumoniae 65 days after infection (14 days after the second immunization), and the vaccine effect was evaluated using the body weight loss and the survival rate as indices.

### (3) Results

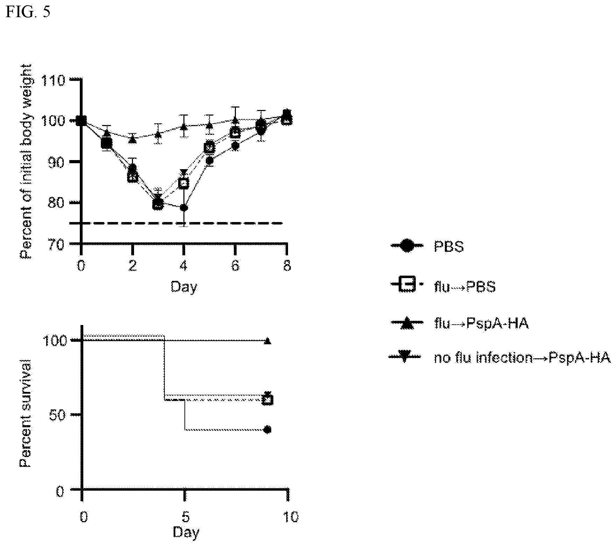

The results of body weight change and survival rate are shown in Fig. 5. When mice were infected with Streptococcus pneumoniae after nasal immunization with PspA-HA, in the group (flu→PspA-HA) in which mice suffering from influenza virus were nasally immunized with PspA-HA, the body weight loss was hardly observed as compared with the control group (flu→PBS) and the group (no flu infection→PspA-HA) in which mice having no history of influenza virus were nasally immunized with PspA-HA. Furthermore, with respect to the survival rate, the survival of all the mice was confirmed in the group in which the mice having a history of influenza virus were nasally immunized with PspA-HA, whereas the survival rate decreased to 40% and 60% in the control group (flu→PBS) and the group in which the mice having no history of influenza virus were nasally immunized with PBS and PspA-HA, respectively (PBS and no flu infection→PspA-HA).

## Claims

1. A fusion protein comprising: (a) a combination of an N-terminal domain of a coronavirus and hemagglutinin and/or neuraminidase; (b) a combination of a receptor binding domain of a coronavirus and PspA; (c) a combination of a G protein of an RS virus and hemagglutinin and/or neuraminidase; or (d) a combination of PspA and hemagglutinin and/or neuraminidase.

2. The fusion protein according to claim 1, wherein the fusion protein contains the combination (a) or the combination (b), and is used as a vaccine antigen against a coronavirus.

3. The fusion protein according to claim 1, wherein the fusion protein contains the combination (c), and is used as a vaccine antigen against an RS virus.

4. The fusion protein according to claim 1, wherein the fusion protein contains the combination (d), and is used as a vaccine antigen against Streptococcus pneumoniae.

5. A vaccine comprising the fusion protein according to any one of claims 1 to 4.

6. The vaccine according to claim 5, wherein the vaccine does not contain an adjuvant.

7. The vaccine according to claim 5, wherein the vaccine contains the combination (a), the combination (c), or the combination (d), and is administered to a subject having an antibody against an influenza virus.

8. The vaccine according to claim 5, wherein the vaccine contains the combination (b), and is administered to a subject having an antibody against Streptococcus pneumoniae.

9. The vaccine according to claim 5, wherein the vaccine is nasally administered.

10. An immunization method comprising administering, to a subject having an antibody against an influenza virus, a vaccine containing a fusion protein containing: (a) a combination of an N-terminal domain of a coronavirus and hemagglutinin and/or neuraminidase; (c) a combination of a G protein of an RS virus and hemagglutinin and/or neuraminidase; or (d) a combination of PspA and hemagglutinin and/or neuraminidase.

11. An immunization method comprising administering, to a subject having an antibody against Streptococcus pneumoniae, a vaccine containing a fusion protein containing (b) a combination of a receptor binding domain of a coronavirus and PspA.
